# EUROPEAN PATENT SPECIFICATION

(11) **EP 1 946 103 B1**
(45) Date of publication and mention of the grant of the patent: **12.01.2011**
(21) Application number: 06818453.0
(22) Date of filing: 10.11.2006
(51) Int. Cl.: G01N 33/50, C12Q 1/00, G01N 1/30

(54) **METHOD FOR ASSAYING ENZYME ACTIVITY IN HISTOLOGICAL SAMPLE**
VERFAHREN ZUM TESTEN VON ENZYMAKTIVITÄT IN EINER HISTOLOGISCHEN PROBE
MÉTHODE POUR DOSER L'ACTIVITÉ D'ENZYME DANS UN ÉCHANTILLON HISTOLOGIQUE

(30) Priority: 10.11.2005 EP 05447252
(43) Date of publication of application: 23.07.2008
(73) Proprietor: PamGene B.V., 5211 's Hertogenbosch (NL)
(72) Inventor: BOENDER, Pieter Jacob Boender, NL-6522 KJ Nijmegen (NL)
(74) Representative: De Clercq, Ann G. Y.
(86) International application number: PCT/EP2006/010776
(87) International publication number: WO 2007/054326

(56) References cited:
- US-B1- 6 251 467
- CURINO ALEJANDRO ET AL: "Detection of plasminogen activators in oral cancer by laser capture microdissection combined with zymography." ORAL ONCOLOGY. NOV 2004, vol. 40, no. 10, November 2004 (2004-11), pages 1026-1032, XP004613704 ISSN: 1368-8375
- YOUSSEF NELLY ET AL: "In situ detection of telomerase enzymatic activity in human hepatocellular carcinogenesis" JOURNAL OF PATHOLOGY, vol. 194, no. 4, August 2001 (2001-08), pages 459-465, XP002385546 ISSN: 0022-3417
- HOFMANN UTA B ET AL: "Coexpression of integrin alphavbeta3 and matrix metalloproteinase-2 (MMP-2) coincides with MMP-2 activation: Correlation with melanoma progression" JOURNAL OF INVESTIGATIVE DERMATOLOGY, vol. 115, no. 4, October 2000 (2000-10), pages 625-632, XP002385547 ISSN: 0022-202X
- HOFMANN U B ET AL: "Matrix metalloproteinases in human melanoma cell lines and xenografts: Increased expression of activated matrix metalloproteinase-2 (MMP-2) correlates with melanoma progression" BRITISH JOURNAL OF CANCER, vol. 81, no. 5, November 1999 (1999-11), pages 774-782, XP002385548 ISSN: 0007-0920
- LARSSON L-I ET AL: "DISTRIBUTION OF UROKINASE-TYPE PLASMINOGEN ACTIVATOR IMMUNOREACTIVITY IN THE MOUSE" JOURNAL OF CELL BIOLOGY, vol. 98, no. 3, 1984, pages 894-903, XP002385618 ISSN: 0021-9525
- MAZAUD SEVERINE ET AL: "Basal membrane remodeling during follicle histogenesis in the rat ovary: contribution of proteinase of the MMP and PA families" DEVELOPMENTAL BIOLOGY, vol. 277, no. 2, 15 January 2005 (2005-01-15), pages 403-416, XP004690157 ISSN: 0012-1606
- SUGINO TAKASHI ET AL: "Telomerase activity in human breast cancer and benign breast lesions: Diagnostic applications in clinical specimens, including fine needle aspirates" INTERNATIONAL JOURNAL OF CANCER, vol. 69, no. 4, 1996, pages 301-306, XP008065383 ISSN: 0020-7136
- VAN BEUNINGEN R ET AL.: "Kinase profiling in a new dimension on PamChip(R) arrays"[Online] 23 March 2004 (2004-03-23), XP002385619 San Francisco Retrieved from the Internet: URL:http://www.pamgene.com/content/pdf/rin ie_kinase.pdf> [retrieved on 2006-06-16] & PAMGENE WEBSITE, [Online] Retrieved from the Internet: URL:http://www.pamgene.com/> [retrieved on 2006-06-16]
- RUSK NICOLE: "When chemistry meets biology. proteases prosper" NATURE METHODS, vol. 2, no. 1, January 2005 (2005-01), page 10, ISSN: 1548-7091
- RUSK NICOLE: "When chemistry meets biology. proteases prosper" NATURE METHODS, vol. 2, no. 1, January 2005 (2005-01), page 10, ISSN: 1548-7091
- [Online] Merriam-Webster Online Dictionary; 27 August 2008 (2008-08-27), "Protease" retrieved from WWW.MERRIAM-WEBSTER.COM
- "Telomerase PCR ELISA. For fast and sensitive detection of telomerase activity." BIOCHEMICA, vol. 4, 1996, pages 7-8,

## Description

### Field of the Invention

The present invention relates to assaying enzyme activity in a biological sample. In particular, the present invention relates to assaying enzyme activity in cell lysates obtained from fixated biological samples.

### Background

Biological samples are being analyzed in various ways for various purposes. Histology is a key component of human and animal disease diagnostics. Typically, biological samples are fixed, embedded, sectioned and stained for the purpose of histological, cytological, hematological or microbiological examination. It is clear that the objective of a fixative is the arrest of autolysis and bacterial decomposition, the stabilization of the cellular and tissue constituents, and the preservation of tissue substances and proteins. A large variety of fixatives is available. The most widely employed universal fixatives are aldehydes such as formaldehyde and gluteraldehyde. For example, formaldehyde, as 4% buffered formaldehyde or 10% buffered formalin is mostly used for routine paraffin embedded sections. Aldehydes form cross-links between proteins, the reaction being mostly with basic amino acid lysine, and other groups such as imino, amido, peptide, guanidyl, hydroxyl, carboxyl, SH and aromatic rings may also be involved. Soluble proteins are fixed to structural proteins and rendered insoluble.

Alternative fixatives include oxidizing agents such as metallic ions and complexes such as osmium tetroxide and chromic acid; and protein-denaturing agents such as acetic acid, methyl alcohol (methanol) and ethyl alcohol (ethanol).

Typically, the use of (chemical) fixatives as described above can be postponed until needed by archiving biological samples after embedding them in an embedding matrix such as, for instance, plastic embedding molds. The examination of microscopic anatomy provides important insights into disease mechanisms, yet the fixation of a biological sample is incompatible with the biochemical analysis of protein function or enzyme activity in that sample because fixation, by its very nature, kills cells and freezes dynamic cell processes and the presence of small diffusible molecules and enzyme activity is lost during chemical fixation.

However, a further study of protein function and analysis of enzymatic reactions in a biological sample that is selected through histological examination, would boost the value of clinical research and *in vitro* diagnostics.

Signal transduction is one of the most important areas of investigation in biological research, and involves many types of interactions. One of the major mechanisms frequently employed by cells to regulate their activity, and in particular to regulate signal transduction processes, involves changes in protein phosphorylation. As many as 1000 protein kinases and 500 protein phosphatases in the human genome are thought to be involved in phosphorylation processes. The targets of phosphorylation encompass a large group of signalling molecules, including enzymes.

The activity of biologically active molecules such as enzymes is also often regulated by direct interactions with other molecules or regulator molecules. The regulation of enzymes controls their activity giving a full range from very fine to coarse control. Many mechanisms for regulating, e.g. inhibiting enzymes are used in living systems.

In general, notwithstanding much progress, the complex system wherein particular substrates are subject to the action(s) of specific enzymes still requires much molecular examination in characterizing still unknown properties of enzymes governing signaling pathways which in turn control cell growth, disease progression and cellular differentiation.

The present invention provides for assaying the activity of subcellular features, in particular enzymes, in respect of the histological diagnosis of a biological sample, bridging the gap between traditional and molecular biology assay data.

The present invention aims at providing methods for assaying enzyme activity of histological examined biological samples.

The present invention aims at correlating histology and enzymatic activity of a biological sample.

In particular, the present invention aims at providing enzyme activity data for a biological sample selected on its histochemical or immuno-histochemical pattern.

The present invention aims at providing methods for screening of enzymatic activity on a multiplex platform.

The present invention aims at providing enzyme assays that can be automated in particular in the pharmaceutical industry as biologically active molecules such as enzymes are often important key players in drug discovery.

The present invention aims at providing methods for differentiation of clinical samples on the basis of enzyme activity in clinical research and in-vitro-diagnostics.

### Summary of the Invention

The present invention provides a novel method for the measurement of hinase activity profiling and fingerprinting in lysates of concomitantly prepared and histologically oxamined samples. In particular, the present invention provides a method for correlating histology and enzymatic activity of a biological sample, the method comprising the steps of:
(i) providing a cryopeserved biological sample, wherein said sample comprises a series of consecutively sliced sections;
(ii) obtaining histological data of a subset of sections of said series of sections
(iii) lysing a second subset of sections of said series of sections to obtain a lysate wherein specific enzyme activities are preserved;
(iv) assaying the lysate of step (iii) for kinase activity with one or more enzyme substrates comprising amino acid sequences immobilized on a microarray; and
(v) correlating the histology and kinase activity of the biological sample.

The methods according to the present invention help in finding disease related pathways at a cell-signalilng level. The present methods are valuable in finding and validating new targets. A target is a molecule or molecule structure of which the activity, when influenced by any means, will have desirable effects on the cells that contain this molecule. A target is a protein or an enzyme which can be used (targeted) by the pharmaceutical industry to develop now drugs. At the same time, this type of enzyme activity fingerprinting can be used as a tool to find predictive efficacy and toxicity biomarker sets which can be used as an aid to clinical trials in the oncology field. The term 'predictive efficacy' relates to the measurement of an effect by e.g. a drug on a target. The term 'toxicity biomarker set' relates to the measurement of side effects of a drug on a non-target. Thus, histopathology and molecular biology data can show indirect effects of a drug treatment. Effects of drugs on other parts of a cell that is not the target often leads to unwanted side effects. The methods of the present invention allow the prediction of these so-called off-target effects so that side effects may be prevented.

### Detailed Description of the Invention

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended clai ms.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The present invention bridges the gap between traditional histochemical staining and microscopic analysis, and molecular biology assay data gathering by providing methods as described herein for assaying the activity of subcellular features, in particular enzymes, in respect of the histological diagnosis on a biological sample.

By 'enzymes' we refer to proteins that are able to modify other proteins. The modified protein - referred to as 'substrate' - might be either another enzyme or any other protein participating in the same signal transduction pathway. Also, nucleic acids, sugars etc may be modified by enzymes.

The most widely used fixatives relate to agents for protein-protein linkage such as formaldehyde and gluteraldehyde. Alternative fixatives include methyl and ethyl alcohol which also alters the structure of proteins, primarily due to the disruption of the hydrophobic bonds which contribute to the maintenance of the tertiary structure.

Contrary to the known irreversible interference of (chemical) fixatives in maintaining proper protein structure, the present inventors surprisingly found that a lysate obtained from a fixated sample, in particular a non-formaline embedded, or, more particularly a cryopreserved biological sample or section, still allowed the measurement of enzymatic activity in the lysate by monitoring its reaction with an appropriate substrate, while the biological characteristics could be defined by standard histological or immuno histochemical procedures.

Within the present specification, the term "cryopreservation" relates to the treatment of a biological sample with a cryoprotectant followed by cooling to a low sub-zero temperature of (typically) -80°C to -196°C (the boiling point of liquid nitrogen). The term "cryoprotectant" and "vitrification solution" are used interchangeably throughout the present specification and refers to a sufficiently non-toxic and sufficiently stable solution to allow viability of a sample to be maintained and ice-formation to be avoided.

Accordingly, within one embodiment of the present invention, a method is provided as described herein, wherein the biological sample, for which a correlation may be sought between the histological data and the enzymatic activity within the corresponding cellular compartments, is treated with a suitable vitrification solution or cryoprotectant prior to cryopreservation. Typically, the vitrification solution prevents cell damage. As such the biochemical reactions that would otherwise lead to cell death is effectively stopped. The vitrification solution allows the conversion of material into a glass-like amorphous solid which is free of any crystalline structure. Concomitantly, most vitrification solutions known in the art also denature the proteins of the sample, thereby preventing further enzymatic analysis. The present inventors know observed that particular cryopreservation still allowed enzymatic analysis of a previously fixated biological sample.

Within one embodiment of the present invention, a method is provided as described herein, wherein a non-formaline chemical fixative is used, in particular an ethanol-based fixative.

Alternatively, particular suitable cryoprotectants or vitrification solutions for use within the present invention are, for example, OCT (Baxter M7148-4), M1 (Lipsahw) or Tissue-Tek^{®} OTC (Sakura Finetechnical Co).

More in particular, within another embodiment of the present invention, a method is provided as described herein wherein said cryoprotectant is Tissue-Tek^{®} OTC (Optimal Cutting Temperature medium, Sakura Finetechnical Co).

Fixed biological samples are routinely embedded in a solid material which will support very thin sectioning. To embed a tissue sample, tissue water is replaced first by solvents, such as alcohol and xylene, and then with a liquid such as melted wax (paraffin) or epoxy solution which can be subsequently solidified by cooling and polymerisation.

Within the present invention, also the cryoprotectant may serve as an embedding medium. Hence, once the biological sample is embedded by treatment with the cryoprotectant, it can be frozen and stored untill needed. From this cryopreserved material, very thin slices may then be prepared by using a sectioning tool such as a microtome or, alternatively, a cryotome which may be as simple as a razor blade, or it may be a complex machine. Sections for routine light microscopy are typically 5-10µm in thickness. Exceptionally thin sections may be less than 2µm thick. For electron microscopy, sections are typically 50-100 nanometers in thickness. The consecutively sliced sections can either undergo standard histological procedures, representing the fixed and embedded biological sample or can undergo the enzymatic assay. Within the present invention, from a first subset of sections, histological data is obtained and from a second subset of sections a lysate is obtained wherein specific enzyme activities may be preserved and detected.

Within the methods of the present invention, a subset of the series of sections that make up the original biological sample is examined through visualisation aided by staining. Most cells are essentially transparent, with little or no intrinsic pigment. Stains are used to confer contrast, to make tissue components visibly conspicuous. The choice for a particular stain will be usually based on the application of particular molecules that can be selectively stained using radioactive labels, enzyme reactions or specific antigen binding.

The procedures of fixing, embedding, sectioning and staining of biological samples are according to the manufacturer's instructions and/or known to the skilled person.

Within one embodiment of the present invention, a method is provided as described herein, wherein the biological sample is paraffin embedded.

Obtaining histological data may be according to the methods as known in the art, including standard immunohistochemistry or *in situ* hybridisation (ISH).

### Lysis of embedded biological material and preservation of selected enzyme activities

Within the present invention, sectioned cryopreserved biological material is either examined using histology tools or lysed to obtain a cell-lysate for further enzymatic analysis.

In biological research, detergents are used to lyse cells and release soluble proteins. Generally, moderate concentrations of mild (i.e., non-ionic) detergents compromise the integrity of cell membranes, thereby facilitating lysis of cells and extraction of soluble protein, often in native form.

Detergents can be denaturing or non-denaturing with respect to protein structure. Denaturing detergents can be anionic such as sodium dodecyl sulfate (SDS) or cationic such as ethyl trimethyl ammonium bromide. These detergents totally disrupt membranes and denature proteins by breaking protein-protein interaction. Non-denaluring detergents can be divided into non-ionic detergents such as Triton^{®} X-100, bile salts such as cholate and zwitterionic detergents such as CHAPS.

Conditions for lysis of biological material or cells are well known in the art.

Within the present invention, the sectioned biological material is lysed under non-donaturing conditions using for example standard Triton-X100 based lysis medium.

The composition of the lysis media, in respect of the enzymatic activity of interest to be monitored, comprises agents that selectively retain specific enzymatic activities while suppressing or eliminating other enzymatic activities of non-interest. For example, enzymatic kinase activity can be measured if the lysis buffer contains specific phosphatase inhibitors such as a mixture containing Triton X-100 and sodium-pyrophosphate, sodiumfluoride and sodiumorthovandate,

### Sample

The present invention is directed to biological samples comprising enzymes as biologically active analyte molecules, allowing the discovery of activities for which the currently known arsenal of enzymes was not evolutionarily selected.

The term 'biological sample' as used herein, refers to a sample obtained from an organism such as human, plant, bacteria, fungl, etc., or from components (e.g., cells) of such an organism. The sample may be of any biological tissue or fluid. Frequently a sample will be a 'clinical sample' which is a sample derived from a patient. Such samples include, but are not limited to, sputum, cerebrospinal fluid, blood, blood fractions such as serum including total serum (e.g., SFC) and plasma, blood cells (e.g., white cells), tissue or fine needle biopsy samples, urine, peritoneal fluid, and pleural fluid, or cells thoro from.

Particularly, the present invention is directed to analysis of kinase activity in a cryopreserved sample. Kinases that may be analysed include, but are not limited to, kinases such as calcium/calmodulin kinase, cyclin-dependent kinases, lipid signaling kinases, mitogen-activated kinases, PDK1-PKB/Akt, PKA, PKC, PKG, non-receptor protein tyrosine kinases, receptor protein tyrosine kinases, serine/threonine kinases.

The methods according to the present invention are equally directed to kinases without a known biologically active function.

### Substrates

A wide variety of different molecules may be used as substrates within the present invention. The term 'substrate' within the present specification refers to an enzyme substrate which is a substance upon which an enzyme acts in a biochemical reaction. The present methods contemplate, as suitable substrates, amino acid sequences including peptides, proteins, enzymes, enzyme substrates, hormone receptors, monoclonal antibodies, polyclonal antibodies, synthetic antibodies, antibody fragments, haptens, and aptamers; nucleic acid sequences including oligonucleotides; and oligosaccharides, The methods according to the present invention are particularly exemplified herein in terms of proteins as substrates.

Accordingly, in one embodiment of the present invention, methods are provided wherein said substrate is chosen from the group comprising amino acid sequences including peptides, proteins, enzymes, hormone receptors, monoclonal antibodies, antibody fragments, and haptens.

Equally useful substrates may be selected from the group comprising agonists and antagonists; toxins and venoms; viral epitopes; hormones (e.g. steroids etc.) and hormone receptors; drugs; lectins; sugars; carbohydrate binding proteins and other interactants.

### Solid porous support

The enzyme substrates useful within the present invention may be immobilized on a solid support. A number of materials suitable for use as support in the present invention have been described in the art. Materials particularly suitable for use as support in the present invention inclucle any type of solid support in the art- More materials particularly suitable for use as support in the present invention include any type of solid porous supports known in the art. The term 'porous support' as used in the present specification refers to a support possessing or full of pores, wherein the term 'pore' refers to a minute opening or microchannel by which matter may be either absorbed or passed through. Particularly, where the pores allow passing-through of matter, the support is likely to be permeable.

Particular useful porous supports as employed in the methods described in the present specification are 3-dimensional porous supports, which allow pressurized movement of fluid, e.g. the sample solution, through its structure. As such, particular useful porous supports as employed in the present methods possess a permeable or flow-through nature.

Accordingly, in one embodiment of the present invention, methods are provided wherein said solid support is a three-dimensional flow-through solid support comprising through-going channels.

A porous solid support, suitable for use within the present invention, may be manufactured out of, for example, a metal, a ceramic metal oxide or an organic polymer. In view of strength and rigidity, a metal or a ceramic metal oxide may be used. Above all, in view of heat resistance and chemicals resistance, a metal oxide may be used. In addition, metal oxides provide a support having both a high channel density and a high porosity, allowing high density arrays comprising different first binding substances per unit of the surface for sample application. In addition, metal oxides are highly transparent for visible light. Metal oxides are relatively cheap that do not require the use of any typical microfabrication technology and, that offers an improved control over the liquid distribution over the surface of the support, such as an electrochemically manufactured metal oxide membrane. Metal oxide membranes having through-going, oriented channels can be manufactured through electrochemical etching of a metal sheet.

Accordingly, in one embodiment of the present invention, methods are provided wherein said solid support is a metal oxide support.

Metal oxide supports or membranes as employed in the methods of the present invention may be anodic oxide films. As well known in the art, aluminium metal may be anodized in an electrolyte to produce an anodic oxide film. The anodization process results in a system of larger pores extending from one face and interconnects with a system of smaller pores extending in from the other face. Pore size is determined by the minimum diameters of the smaller pores, while flow rates are determined largely by the length of the smaller pores, which can be made very short. Accordingly, such membranes may have oriented through-going partially branched channels with well-controlled diameter and useful chemical surface properties. Useful thicknesses of the metal oxide supports or membranes as employed in the methods and apparatuses of the present invention may for instance range from 50 µm to 150 µm (including thicknesses of 60, 70, 80, 90, 100, 110, 120, 130 and 140 µm). A particular suitable example of support thickness is 60 µm.

A suitable support pore diameter ranges from 150 to 250 nm including 160, 170, 180, 190, 200, 210, 220, 230 and 240 nm. A particular suitable example of pore diameter is 200 nm. These dimensions are not to be construed as limiting the present invention.

Accordingly, in a further embodiment of the present invention, methods are provided wherein said metal oxide support is an aluminium oxide support.

Advantageously, metal oxide membranes as described herein are transparent, especially if wet, which allows for assays using various optical techniques. WO 99/02266 which discloses the Anopore^{™} porous membrane or support is exemplary in this respect

Further, a positive or negative pressure may be applied to the arrays in order to pump e.g. a sample solution dynamically up and down through the support pores. Said dynamical pumping allows immediate removal and ability to perform real-time detection of generated products from a reaction which takes place within the pores of the support by fast binding of said generated products to the support pore walls.

Accordingly, in one embodiment of the present invention, methods are provided wherein said assaying of the lysate for enzymatic reaction with one or more substrates is by dynamic incubation.

Pumping the lysate solution dynamically up and down through the support pores may enhance diffusion of enzyme to substrate molecules, may increase the rate of accumulation of enzyme specifically bound to a binding substrate partner and accordingly may result in increased opportunity for rare high-affinity enzymes to bind to a substrate. Further, bound enzymes may be replaced by higher affinity enzymes over an extended period of exposure to the substrate, thus allowing for selection of those enzymes with the highest affinity for a given substrate.

In a further embodiment, methods are provided wherein said dynamic incubation comprises subjecting said porous support to at least one cycle of forward and backward flow of lysate across said through-going channels. Said forward and backward flow is established by application of alternating positive and negative pressure on the lysate relative to the surrounding ambient pressure which will force the lysate to flow in up and down direction through the porous support. As such, a positive pressure within the context of the present invention relates to a pressure above atmospheric pressure. A negative pressure within the context of the present invention relates to a vacuum or a pressure below atmospheric pressure.

The duration of one cycle of forward and backward flow of sample across the porous substrate may be comprised between 10 min and 1 sec. Usually, duration of one cycle is comprised between 5 min and 10 sec. More usually, duration of one cycle is comprised between 2 min and 30 sec. A particular suitable example of duration of a single cycle of forward and backward flow is 60 sec.

An optimum flow rate is such that the residence time of the sample molecules near the immobilized molecules is sufficient to generate interaction events of measurable quantities in the shortest possible time.

Methods according to the present invention make use of a three-dimensional multiplex platform with interconnecting pores that enables real-time kinetics. This technology is scalable and enables parallel analysis of many hundreds of samples on many hundreds of different substrates. The methods according to the present invention allow for continuous as well discontinuous monitoring of interactions between enzyme and substrate. Accordingly, the methods according to the present invention allow the determination of enzyme-substrate interaction in real time.

Within the present invention, substrates may be attached or adhered to the surface of a porous solid support including attachment or adherence to the inner surface of said support.

Substrates may be immobilized either covalently (e.g., utilizing single reactive thiol groups of cysteine residues) or non-covalently but specifically (e.g., via immobilized antibodies, the biotin/streptavidin system, and the like), by any method known in the art. Further examples of the various methods that are available to attach target molecules to porous supports include but are not limited to biotin-ligand non-covalently complexed with streptavidin, S-H-ligand covalently linked via an alkylating reagent such as an iodoacetamide or maleimide, amine-ligand covalently linked via an activated carboxylate group (e.g., EDAC coupled, etc.), phenylboronic acid (PBA)-ligand complexed with salicylhydroxamic acid (SHA), and acrylic linkages allowing polymerization with free acrylic acid monomers to form polyacrylamide or reaction with SH or silane surfaces. More specifically, immobilization of proteins may be accomplished through attachment agents selected from the group comprising cyanogen bromide, succinimides, aldehydes, tosyl chloride, avidin-biotin, photo-crosslinkable agents including hetero bi-functional crosslinking agents such as N-[y-maleimidobutyryloxylsuccinimide ester (GMBS), epoxides, and maleimides. Antibodies may be attached to a porous support by chemically crosslinking a free amino group on the antibody to reactive side groups present within the support. For example, antibodies may be chemically cross-linked to a support that contains free amino, carboxyl, or sulfur groups using glutaraldehyde, carbo-di-imides, or hetero bi-functional agents such as GIVMS as cross-linkers.

Accordingly, in one embodiment of the present invention, methods are provided wherein the substrates are immobilized on the inner surface of through-going channels within a solid support.

Substrates may be immobilized on a solid support within spatially predefined regions, i.e. within particular spots. The terms 'predefined region' or 'spot' relate to individually, spatially addressable positions on a solid support.

### Detection

Enzyme-substrate interactions may be detected in a number of ways. Enzyme-substrate interactions may include a binding event which may be visualised by detection of the substrate molecule which may be a labelled protein. Said protein may be labelled either directly or indirectly, with e.g. an antibody, or a dye etc. Alternatively, a reaction event may be visualised via generation of fluorescence proximal to the place where interaction takes place (FRET, removal of quenching (such as with molecular beacons)). Further, detection may be by generation of a signal by enzymatic action or reduction in signal intens ity by the presence of an unbound compound (competition principle).

Means for detecting signals in general are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with an enzyme substrate and detecting the reaction product produced by the action of the enzyme on the substrate, and colorimetric labels are detected by simply visualizing the coloured label. Further detection means are for example (micro-)calorimetry and (light)-microscopy.

Particular useful detection of enzyme-substrate interaction(s) within the methods of the present invention is by luminescence or fluorescence microscopy, regular light microscopy, electron microscopy, electro-chemiluminiscence (ECL), UV/VIS absorbance, microcalorimetry and radiometry. Said fluorescence microscopy may be time-resolved fluorescence and fluorescent correlation spectroscopy (FCS).

Similar to fluorescence, also phosphorescence provides a suitable detection means. Phosphorescence relates to a quasi-stable electron excitation state involving a change of spin state (intersystem crossing) which decays only slowly. It is similar to fluorescence, but the species is excited to a metastable state from which a transition to the initial state is forbidden.

Detection of enzyme-substrate interactions may also be accomplished by multi-step detection practices. Said practices may be, by way of example and not limitation, sandwich assays as are well-known in the art and enzymatic conversions into a detectable product.

The present invention also contemplates the monitoring of more than one enzyme-substrate interaction (i.e., multiplexing) by for example looking at fluorescence at different wavelengths by using e.g. CY3 and CY5 dyes, or by simultaneously employing different methods for detection.

### Applications

The methods according to the present invention are useful in a number of applications.

In one embodiment, the present invention provides for the use of methods as described herein for drug screening.

In another embodiment, the present invention provides for the use of methods as described herein for assessing enzyme selectivity for a substrate in respect of the histological pattern of a given biological sample.

In another embodiment, the present invention provides for the use of methods as described herein for differentiation of clinical samples on the basis of enzyme activity in clinical research and *in-vitro*-diagnostics.

### EXAMPLES

The following example of the invention is exemplary and should not be taken as in any way limiting.

### Example 1: Assaying kinase activity in a Tissue-Tec^{®} preserved biological sample

Frozen brain biopsies from patients diagnosed as suffering from an ependymome or an astrocytome were embedded in a commercially available polyvinyl alcohol, polyethylene glycol based embedding medium (Tissue-Tek^{®} OTC, Sakura Finetek Europe B.V.), snap frozen in liquid nitrogen and stored at -70°C. When needed, the frozen specimens were sliced in sections of 10 µm using a cryostat. A subset of the sections was mounted on glass slides and fixed in acteone for 5 minutes at room temperature and stained with standard hematoxylin/eosin (HE) to investigate the morphological features of the biopsies. Another subset of 6 slices, representing approximately 100 µg of sectioned material, was lysed in a 50 µl buffer solution by pipetting the buffer solution containing the sections up and down a few times, wherein the buffer solution comprises 20 mM TRIS-HCl (pH 7.5), 150 mM NaCl, 1 mM EDTA, 1 mM EGTA, 1% Triton X-100, 2.5 mM sodium pyrophosphate, 1 mM beta-glycerophosphate, 1 mM sodiumvanadate, 1 mM sodiumfluoride, 1 ug/ml leupeptin, 1 µg/ml aprotinin and 1 mM PMSF. This buffer solution was freshly prepared before use. The lysed material was subsequently centrifuged on high speed for 10 minutes at 4°C to remove debris. The supernatant was frozen at -80°C.

5 µl of the lysate (representing approximately 10 µg) was used for incubation on a flow-through microarray (PamChip^{®}96, PamGene International B.V., The Netherlands) by adding it to a solution containing 50mM Tris-HCl pH 7.5, 10mM MgCl2, 1mM EGTA, 2mM DTT, 0.01% Brij35 detergent (Sigma Cat. No. 430AG-6), 1*BSA (from 100*BSA. New England Biolabs, cat nr B9001S), 400uM ATP (Sigma, cat.nr. A7699) and 0.25 µl of a FITC conjugated monoclonal anti-phosphotyrosine antibody (Exalpha, cat nr X1017, 1mg/ml) on ice and adding this mixture immediately to a PamChip^{®}96. The microarray contained 144 16-mers peptides, representing various tyrosine kinase recognition motifs.

The assessed enzymatic reaction related to the addition of a phosphate-group to the peptides on the microarray by kinases present in the mixture. The reaction mixture was dynamically incubated by flowing the mixture up and down trough the pores of the microarray during 60 minutes, one cycle of up-and-down flow per minute. The reaction was monitored by taking pictures every five cycles at the appropriate wavelength. A differential increase in phosphorylation on the various peptides could be discerned, proving the activity of various kinases in the incubation mixture.

The resulting phosphorylation patterns were correlated with the biological features determined with the standard HE staining.

## Claims

1. Method for correlating histology and hinase activity of a biological sample, the method comprising the steps of:
(i) providing a cryopreserved biological sample, wherein said sample comprises a series of consecutive sliced sections;
(ii) obtaining histological data of a first sub-set of sections of said series of sections;
(iii) lysing a second sub-set of sections of said series of sections to obtain a lysate wherein specific enzyme activities are preserved;
(iv) assaying the lysate of step (iii) for kinase activity with one or more enzyme substrates comprising amino acid sequences, immobilized on a microarray; and
(v) correlating the histology and kinase activity of the biological sample.

2. Method according to claim 1, wherein said biological sample in step (i) was treated with a cryoprotectant.

3. Method according to claim 2, wherein said cryoprotectant is an OCT (Optimal Cutting Temperature) compound.

4. Method according to any of claims 1 to 3, wherein said substrate comprises amino acid sequences chosen from the group including peptides, proteins, enzymes, hormone receptors, monoclonal antibodies, antibody fragments, and haptens.

5. Method according to any of claims 1 to 4, wherein said microarray is a three-dimensional flow-through solid support comprising through-going channels.

6. Method according to Claim 5, wherein said substrates are immobilized on the inner surface of the through-going channels within the solid support.

7. Method according to claim 5 or 6, wherein said solid support is a metal oxide support.

8. Method according to claim 7, wherein said metal oxide support is an aluminium oxide support.

9. Method according to any of the claims 1 to 8, wherein said assaying of the lysate for enzymatic reaction with one or more substrates is by dynamic incubation, wherein said dynamic incubation comprises subjecting the porous support to at least one cycle of forward and backward flow of lysate across the through-going channels.

10. Use of a method according to any of claims 1 to 9, for differentiation of clinical samples on the basis of enzyme activity in clinical research and *in-vitro-*diagnostics.

11. Use according to claim 10, for drug screening.

## Patentansprüche

1. Verfahren zum Korrelieren von Histologie und Kinase-aktivität in einer biologischen Probe, wobei das Verfahren die folgenden Schritte umfasst:
(i) Bereitstellen einer kryokonservierten biologischen Probe, wobei die Probe eine Serie aufeinanderfolgender Schnitte umfasst;
(ii) Erhalten der histologischen Daten von einer ersten Teilmenge an Schnitten von den Schnittserien;
(iii)Lysieren einer zweiten Teilmenge der Schnittserien, um ein Lysat zu erhalten, in dem spezifische Enzymaktivitäten erhalten sind;
(iv) Untersuchen des Lysats von Schritt (iii) auf Kinaseaktivität mit einem oder mehreren Enzymsubstraten, die Aminosäuresequenzen umfassen, die auf einem Mikroarray immobilisiert sind; und
(v) Korrelieren der Histologie und der Kinaseaktivität von der biologischen Probe.

2. Verfahren nach Anspruch 1, wobei die biologische Probe in Schritt (i) mit einem Gefrierschutzmittel behandelt wurde.

3. Verfahren nach Anspruch 1, wobei das Gefrierschutzmittel ein OCT-(Optimal Cutting Temperature)Compound-Einbett-medium ist.

4. Verfahren nach einem der Ansprüche 1 bis 3, wobei das Substrat Aminosäuresequenzen umfasst, die aus der Gruppe ausgewählt sind, die Peptide, Proteine, Enzyme, Hormonrezeptoren, monoklonale Antikörper, Antikörperfragmente und Haptene einschließt.

5. Verfahren nach einem der Ansprüche 1 bis 4, wobei der Mikroarray ein festes dreidimensionales, durchströmtes Trägermaterial ist, das durchgehende Kanäle umfasst.

6. Verfahren nach Anspruch 5, wobei die Substrate auf der Innenfläche der durchgehenden Kanäle innerhalb des festen Trägermaterials immobilisiert sind.

7. Verfahren nach Anspruch 5 oder 6, wobei das feste Trägermaterial ein Metalloxid-Trägermaterial ist.

8. Verfahren nach Anspruch 7, wobei das Metalloxid-Trägermaterial ein Aluminiumoxid-Trägermaterial ist.

9. Verfahren nach einem der Ansprüche 1 bis 8, wobei die Untersuchung von dem Lysat auf enzymatische Reaktionen mit einem oder mehreren Substraten durch eine dynamische Inkubation stattfindet, wobei die dynamische Inkubation das Unterwerfen des porösen Trägermaterials für mindestens einen Zyklus von Vor- und Rücklauf von dem Lysat durch die durchgehenden Kanäle hindurch umfasst.

10. Verwendung von einem Verfahren nach einem der Ansprüche 1 bis 9 zur Differenzierung von klinischen Proben auf der Basis der Enzymaktivität in der klinischen Forschung und der In-Vitro-Diagnostik.

11. Anwendung nach Anspruch 10 zum Wirkstoffscreening.

## Revendications

1. Procédé de corrélation de l'histologie et de l'activité kinase d'un échantillon biologique, le procédé comprenant les étapes consistant à:
(i) fournir un échantillon biologique cryoconservé, dans lequel ledit échantillon comprend une série de sections tranchées consécutives;
(ii) obtenir des données histologiques d'un premier sous-ensemble de sections de ladite série de sections;
(iii) lyser un deuxième sous-ensemble de sections de ladite série de sections pour obtenir un lysat dans lequel des activités enzymatiques spécifiques sont conservées;
(iv) doser le lysat de l'étape (iii) pour l'activité kinase avec un ou plusieurs substrats enzymatiques comprenant des séquences d'acides aminés, immobilisés sur un microréseau; et
(v) corréler l'histologie et l'activité kinase de l'échantillon biologique.

2. Procédé selon la revendication 1, dans lequel ledit échantillon biologique de l'étape (i) a été traité avec un cryoprotecteur.

3. Procédé selon la revendication 2, dans lequel ledit cryoprotecteur est un composé TDO (température de découpe optimale).

4. Procédé selon l'une quelconque des revendications 1 à 3, dans lequel ledit substrat comprend des séquences d'acides aminés choisies parmi le groupe comprenant des peptides, des protéines, des enzymes, des récepteurs hormonaux, des anticorps monoclonaux, des fragments d'anticorps, et des haptènes.

5. Procédé selon l'une quelconque des revendications 1 à 4, dans lequel ledit microréseau est un support solide de transfert en trois dimensions comprenant des canaux étendus.

6. Procédé selon la revendication 5, dans lequel lesdits substrats sont immobilisés sur la surface interne des canaux étendus au sein du support solide.

7. Procédé selon la revendication 5 ou la revendication 6, dans lequel ledit support solide est un support d'oxyde métallique.

8. Procédé selon la revendication 7, dans lequel ledit support d'oxyde métallique est un support d'oxyde d'aluminium.

9. Procédé selon l'une quelconque des revendications 1 à 8, dans lequel ledit dosage du lysat pour une réaction enzymatique avec un ou plusieurs substrats est par incubation dynamique, dans lequel ladite incubation dynamique comprend l'étape consistant à soumettre le support poreux à au moins un cycle d'écoulement de lysat ascendant et descendant sur les canaux étendus.

10. Utilisation d'un procédé selon l'une quelconque des revendications 1 à 9, pour la différenciation d'échantillons cliniques sur la base de l'activité enzymatique dans la recherche clinique et dans des diagnostics *in-vitro.*

11. Utilisation selon la revendication 10, pour le dépistage de drogue.
